# EUROPEAN PATENT SPECIFICATION

(11) **EP 1 147 211 B1**
(45) Date of publication and mention of the grant of the patent: **17.06.2009**
(21) Application number: 00914444.5
(22) Date of filing: 27.01.2000
(51) Int. Cl.: C12P 21/00, C12N 15/09, C12N 15/70, C07H 21/04, C12N 15/67

(54) **HIGH LEVEL EXPRESSION OF A HETEROLOGOUS PROTEIN HAVING RARE CODONS**
HOHE EXPRESSION EINES HETEROLOGEN PROTEINS MIT SELTENEN CODONS
EXPRESSION HAUT NIVEAU D'UNE PROTEINE HETEROLOGUE A CODONS RARES

(30) Priority: 27.01.1999 US 117355 P
(43) Date of publication of application: 24.10.2001
(73) Proprietor: Stratagene California, La Jolla, CA 92037 (US); Carstens, Carsten-Peter, La Jolla, CA 92037 (US)
(72) Inventor: CARSTENS, Carsten-Peter, San Diego, CA 92130 (US)
(74) Representative: Zimmer, Franz-Josef
(86) International application number: PCT/US2000/002002
(87) International publication number: WO 2000/044926

(56) References cited:
- EP-A- 0 373 365
- EP-A- 0 835 938
- MAKRIDES: "Strategies for Achieving High-Level Expression of Genes in Escherichia coli" MICROBIOLOGICAL REVIEWS, AMERICAN SOCIETY FOR MICROBIOLOGY, WASHINGTON, DC, US, vol. 60, no. 3, 1 September 1996 (1996-09-01), pages 512-538, XP002095235 ISSN: 0146-0749
- KIM ET AL.: 'Overexpression of archaeal proteins in Escherichia Coli' BIOTECHNOLOGY, vol. 20, no. 3, March 1998, pages 207 - 210, XP002926787
- BRINKMANN ET AL.: 'High-level expression of recombinant genes in Escherichia coli is dependent on the availability of the dnaY gene product' GENE, vol. 85, 21 December 1989, pages 109 - 114, XP002926788
- DEL TITO JR. ET AL.: 'Effects of a Minor Isoleucyl tRNA on Heterologous Protein Translation in Escherichia coli' JOURNAL OF BACTERIOLOGY, vol. 177, no. 24, December 1995, pages 7086 - 7091, XP002926789
- KANE J.F.: 'Effects of rare codon clusters on high-level expression of heterologous proteins in Escherichia coli' CURRENT OPINION IN BIOTECHNOLOGY, vol. 6, no. 5, October 1995, pages 494 - 500, XP002926790

## Description

### FIELD OF THE INVENTION

The invention is related to the area of expression of heterologous proteins in bacteria. In particular it is related to the field of codon usage.

### BACKGROUND OF THE INVENTION

Expression of heterologous proteins in bacteria such as *E. coli* has become a standard procedure in most molecular biology laboratories and a cornerstone of production in the biotechnology industry. The most frequent problems encountered in bacterial expression systems are the insolubility of the induced protein and the poor efficiency of expression. Expression of heterologous proteins in bacteria can be seriously compromised due to the different codon preference displayed in other organisms. The codon frequencies in *E.coli* and other organisms are compared in Table 1.

Forced high level expression of a gene that uses a codon rarely utilized in the host bacterial cell can lead to depletion of the cognate tRNA species and subsequent stalling of the translation process (Kane, J.F., "Effects of rare codon clusters on high-level expression of heterologous proteins in Escherichia coli, " Curr. Opin. Biotechnol. 6:494-500 (1995); Bonekamp, F., Andersen, H.D., Christensen, T., Jensen, K.F, "Codon-defined ribosomal pausing in Escherichia coli detected by using the pyre attenuator to probe the coupling between transcription and translation," Nucleic Acids Res. 13:4113-23 (1985)). Stalling of the translation process may result in premature degradation of mRNA (Deana, A., Ehrlich, R., Reiss, C., "Silent mutations in the Escherichia coli ompa leader peptide region strongly affect transcription and translation in vivo," Nucleic Acids Res. 26:4778-4782 (1998)).

Analysis of positional effects of rare codons on protein expression in *E. coli* has demonstrated that these codons are a particular problem if the same codon appears frequently, consecutively, and close to the N-terminus (Rosenberg, A.H., Goldman, E., Dunn J.J., Studier, F.W., Zubay, G., "Effects of consecutive AGG codons on translation in Escherichia coli, demonstrated with a versatile codon test system," J. Bacteriol. 175:716-22 (1993); Goldman, E., Rosenberg, A.H., Zubay, G., Studier, F.W., "Consecutive low-usage leucine codons block translation only when near the 5' end of a message in Escherichia coli, " J. Mol. Biol. 245:467-73 (1995); Degryse, E. , "Influence of the second and third codon on the expression of recombinant hirudin in E. coli, " FEBS Lett. 269: 244-6 (1990)).

The effects of rare *E.coli* codons on high level expression are particularly well documented in the case of the arginine codons AGA and AGG, which are the rarest codons in *E. coli* (reviewed in Kane (1995) supra). However, other codons such as CGA (arg), CUA (leu), AUA (ile), CCC (pro), and the glycine codons GGA and GGG also have been shown to affect protein expression (Kane (1995) supra; Rosenberg et al., (1993) supra; Goldman et al., (1995) supra; Del Tito, B.J. Jr., Ward, J.M., Hodgson, J., Gershater, C.J., Edwards, H., Wysocki, L.A., Watson, F.A,. Sathe, G., Kane, J.F., "Effects of a minor isoleucyl tRNA on heterologous protein translation in Escherichia coli, " J. Bacteriol. 177:7086-91 (1995)).

In addition to effects on yield, the quality of the protein can be affected by the presence of rare codons. Stalling during translation can lead to frameshifts or skipping of a particular codon (Spanjaard, R.A., Chen, K., Walker, J.R., van Duin, J., "Frameshift suppression at tandem AGA and AGG codons by cloned tRNA genes: assigning a codon to argu tRNA and T4 tRNA(Arg)," Nucleic Acids Res. 18:5031-6 (1990); Kane J.F., Violand, B.N., Curran, D.F., Staten, N.R., Duffin, K.L., Bogosian, G., "Novel in-frame two codon translational hop during synthesis of bovine placental lactogen in a recombinant strain of Escherichia coli, " Nucleic Acids Res. 20:6707-12 (1992)).

Another potentially significant problem is the misincorporation of lysine for arginine at AGA or AGG codons (Calderone, T.L., Stevens, R.D., Oas, T.G., "High-level misincorporation of lysine for arginine at AGA codons in a fusion protein expressed in Escherichia coli," J. Mol. Biol. 262:407-12 (1996)). Misincorporation rates can reach 43% at a given residue (Forman, M.D., Stack, R.F., Masters, P.S., Hauer, C.R., Baxter, S.M., "High level, context dependent misincorporation of lysine for arginine in Saccharomyces cerevisiae a1 homeodomain expressed in Escherichia coli, " Protein Sci. 7:500-3 (1998)), which may cause problems if the protein is used for structural analysis.

Expression of E.coli genes for rare codons in bacterial host cells has been found to increase the expression of heterologous proteins requiring the rare codons. Several laboratories have shown that transfection of host cells with the *argU* gene, which encodes a tRNA species utilizing the rare AGA/AGG codons, together with a gene for a recombinant protein requiring these codons can increase heterologous protein expression. Brinkmann et al., Gene 85:109-14 (1989); Hua et al., Biochem. Mol. Biol. Int. 32:537-43 (1994); Chen et al., Genes Dev. 8:2641-52 (1994); and Garcia et al., Ann. N.Y. Acad. Sci. 782:79-86 (1996). A similar effect was observed for transfection with the *ileX* gene, which utilizes the rare isoleucine codon AUA. Del Tito, Jr., et al., J. Bacteriol. 177:7086-7091 (1995). Coexpression with both *argU* and *ileX* genes has also been shown to increase the expression of certain bacterial and viral recombinant proteins. Del Tito, Jr., et al., supra; Kim et al., Biotech. Lett. 20:207-210 (1998). Overexpression of tRNA genes can be deleterious to the host cell, however. Rojiani et al., Proc. Nat. Acad. Sci. U.S.A. 87:1511-1515 (1990); Sharp et al., Nucleic Acids Res. 14:7737-7749 (1986).

There remains a need in the art for methods and reagents which permit high level expression of heterologous recombinant proteins, particularly eukaryotic proteins, whose expression is limited by codon usage in the host cell. Furthermore, there is a need for host cells and methods of making them which can overcome poor usage of several rare codons simultaneously.

### SUMMARY OF THE INVENTION

It is an object of the invention to provide methods and reagents which make possible the high level expression of heterologous recombinant proteins whose codon usage differs from that of the host cells. This and other objects of the invention are provided by one or more of the embodiments described below.

The present invention relates to a pACYC-LIC nucleic acid vector comprising sequences encoding *E*. *coli argU, ileY* and *leuW* tRNAs. Preferably said sequences encoding said *E*. *coli* tRNAs are present in the order *argU, ileY, leuW.*

In a further embodiment the vector described above comprises a *tet* promoter. Preferably said *tet* promoter drives expression of the sequence encoding said tRNAs.

In a further embodiment of the invention, said sequence encoding *E*. *coli argU* tRNA comprises the sequence between base pairs 8041 and 8260 of GenBank Accession No. AE000159.

In a further embodiment of the invention, in the vector described above, said sequence encoding *E. coli ileY* tRNA comprises the sequence between base pairs 7741 and 7950 of GenBank Accession No. AE000350.

In a further embodiment of the invention, in the vector described above, said sequence encoding *E*. *coli leuW* tRNA comprises the sequence between base pairs 241 and 378 of GenBank Accession No. J01713.

In a further aspect, of the the present invention, the invention relates to a nucleic acid comprising a promoter operably linked to sequences encoding *E. coli argU*, *ileY* and *leuW* tRNAs. Preferably said sequences encoding *E*. *coli* tRNAs are present in the order *argU*, *ileY, leuW.*

In a further embodiment of this aspect of the invention said promoter is the *tet* promoter.

Preferably, in the nucleic acid according to the invention the tRNA genes contain their endogenous promoter.

In a further embodiment of this aspect of the invention, said sequence encoding *E*. *coli argU* tRNA comprises the sequence between base pairs 8041 and 8260 ofGenBank Accession No. AE000159.

In a further embodiment of this aspect of the invention, said sequence encoding *E*. *coli ileY* tRNA comprises the sequence between base pairs 7741 and 7950 ofGenBank Accession No. AE000350.

In a further embodiment of this aspect of the invention, said sequence encoding *E*. *coli leuW* tRNA comprises the sequence between base pairs 241 and 378 ofGenBank Accession No. J01713.

Further described herein is a host cell comprising a recombinant DNA molecule which comprises an array of three or more tRNA genes. The tRNA genes correspond to codons that are rarely used in the host cell. In some versions of this embodiment, the tRNA genes correspond to codons present in a gene from an organism other than the host cell. One or more of the tRNA genes can be heterologous to the host cell. The host cell can further comprise a recombinant DNA molecule which comprises a gene encoding a protein of interest.

Preferably, the array of tRNA genes is operatively associated with a transcription control element operative in the host cell. For example, the transcription control element can be activated by isopropylthio-β-galactoside (IPTG), which activates transcription of the tRNA genes. Transcription of the tRNA genes can be controlled by a promoter for T7 RNA polymerase.

The host cell may be protease deficient; for example, the host cell can be deficient in Lon and OmpT proteases. In some host cells of this embodiment, the recombinant DNA molecule comprises a medium to low copy number vector. The medium to low copy number vector can comprise a particular array of tRNA genes. For example, the array can comprise the *E*. *coli* genes *argU* and *ileY*. The array can also comprise the *E*. *coli* genes *argU, ileY*, and *leuW*. The array can also comprise the *E*. *coli* genes *argU, ileY, leuW*, and *proL*. The array can comprise the *E. coli* genes *argU* and *proL*.

Some host cells are bacterial cells, for example *E*. *coli* cells. Some *E*. *coli* cells of this embodiment have the Hte (high transformation efficiency) phenotype. Some *E. coli* cells of this embodiment are EndA1 deficient, and some are RecA positive.

Another embodiment of the invention provides a vector as disclosed above that replicates in a host cell. The vector comprises an array of three or more tRNA genes which correspond to codons that are rarely used in said host cell. Some vectors of this embodiment comprise a restriction endonuclease site located between the coding sequences of any two tRNA genes.

Further disclosed herein is a method of producing a protein of interest. The method comprises the step of culturing a host cell that comprises a recombinant DNA molecule. The recombinant DNA molecule comprises an array of three or more tRNA genes which correspond to codons that are rarely used in the host cell. The codons are present in the gene for the protein of interest. The conditions of culturing the host cell are sufficient to produce the protein of interest.

Further disclosed herein is a kit comprising a vector, a host cell, and packaging materials therefor. The vector replicates in the host cell and comprises an array of three or more tRNA genes corresponding to codons which are rarely used in the host cell. In some kits of this embodiment, the vector comprises a restriction endonuclease site located between the coding sequences of any two tRNA genes.

Further disclosed herein is a host cell comprising a recombinant DNA molecule which comprises a set of two tRNA genes. The set does not consist of the pair of *E*. *coli* genes *argU* and *ileX*. The tRNA genes correspond to codons that are rarely used in the host cell.

In some host cells, the set consists of tRNA genes that specify codons for arginine and proline; for example, the set can comprise the *E*. *coli* tRNA genes *argU* and *proL.*

In some host cells, the set consists of tRNA genes that specify codons for arginine and leucine or arginine and glycine.

In some host cells, the set consists of tRNA genes that specify codons for proline and leucine, proline and isoleucine, or proline and glycine.

In some host cells, the set comprises tRNA genes that specify codons for leucine and isoleucine or leucine and glycine.

In some host cells, the set comprises tRNA genes that specify codons for isoleucine and glycine.

A further embodiment of the invention provides a vector as described above that replicates in a host cell. The vector comprises an array of tRNA genes which correspond to codons that are rarely used in said host cell. The array does not consist of only the pair of *E*. *coli* genes a*rgU* and *ileX.*

Further disclosed herein is a method of producing a protein of interest. The method comprises the step of culturing a host cell that comprises a recombinant DNA molecule. The recombinant DNA molecule comprises an array of tRNA genes which correspond to codons that are rarely used in the host cell, where the array does not consist of only the pair of *E*. *coli* genes *argU* and *ileX*. The codons are present in the gene for the protein of interest. The conditions of culturing the host cell are sufficient to produce the protein of interest.

Further disclosed herein is a kit comprising a vector, a host cell, and packaging materials therefor. The vector replicates in the host cell and comprises an array of tRNA genes corresponding to codons which are rarely used in the host cell, and where the array does not consist of the pair of *E. coli* genes *argU* and *ileX.*

Further features and advantages of the invention will become more fully apparent in the following description of the embodiments and drawings thereof, and from the claims.

### BRIEF DESCRIPTION OF THE DRAWINGS

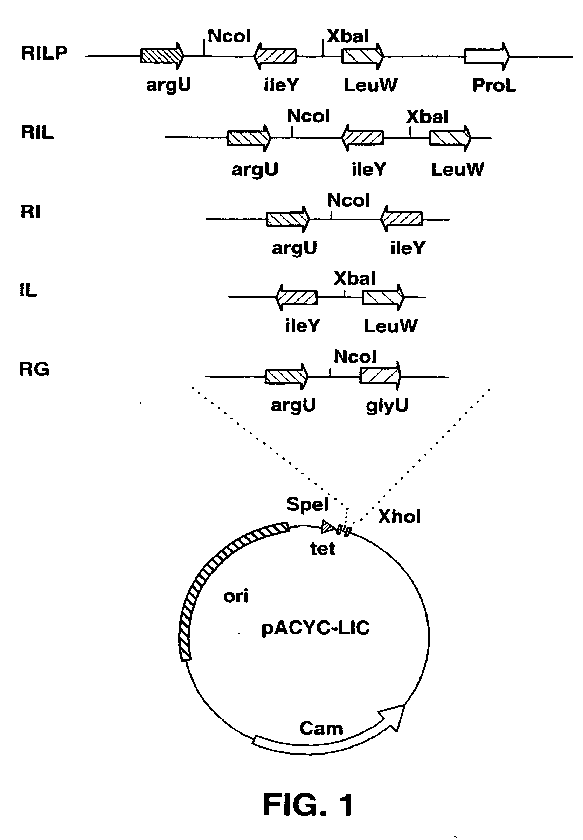

Figure 1 displays pACYC-LIC expression vectors for *E. coli* tRNA gene arrays. All tRNA gene arrays were cloned between the *Spe*I and *Xho*I sites of pACYC-LIC except for the RG array, which was cloned by ligation independent cloning between the LIC sites of pACYC-LIC, indicated by boxes flanking the *Spe*I and *Xho*I sites. The tRNA genes were amplified by PCR from *E.coli* K12 genomic DNA. Arrays were generated by ligation of the individual tRNA fragments and re-amplification using flanking primers for the specific array. The restriction sites demarcate the junctions between tRNA gene fragments. Expression is driven by the tetracycline resistance gene promoter; however, each fragment may contain its own promoter. pACYC-based plasmids are medium to low copy number plasmids compatible with colE1 based plasmids.

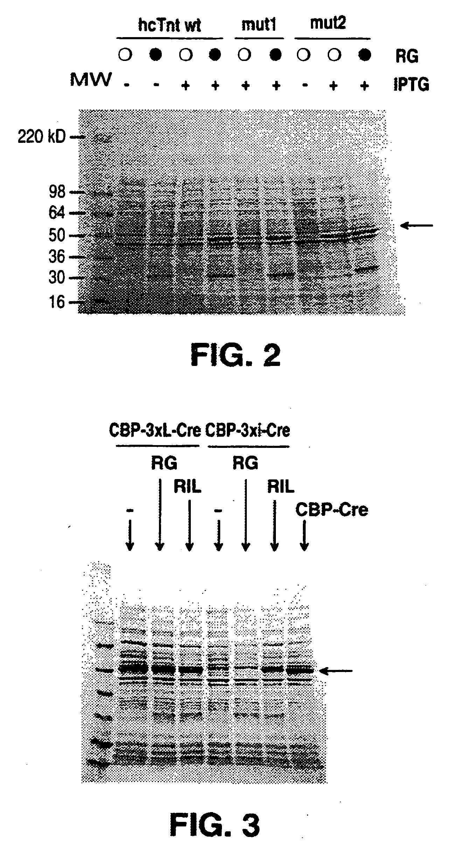

Figure 2 demonstrates rescue of the expression of a derivative of the CBP-Cre fusion protein by introduction of the *ileY* gene. Cultures of BL21gold DE3 cells containing T7-driven vectors for a CBP/Cre-recombinase fusion gene with either three rare leucine (CBP-3xL-Cre, codon CUA) or isoleucine codons (CBP-3xL-Cre, codon AUA) at the 5' end of the gene, and the indicated pACYC-based tRNA expression vectors were induced at mid-log phase for 1h with 1mM IPTG. Fifteen µl of whole cell lysate were loaded on a 4-20% PAGE gel and stained with Coomassie blue. The position of the induced fusion protein is indicated by an arrow. A sample of CBP-Cre without any extra codons was added as a reference. The lanes labeled with RG contain an expression vector for the tRNA genes *argU* and *glyU*. The lanes labeled RIL contain an expression vector for the *argU, ileY* and *leuW* tRNA genes.
Figure 3 demonstrates the functionality of the *proL* gene in an RILP array. Cultures of BL21goldDE3 strains containing pACYC-based vectors expressing copies of the indicated *E.coli* tRNA genes and the T7-driven expression vectors for human cardiac troponin T (*argU*-dependent) and the Cre-recombinase/CBP fusion genes CBP-exi-Cre (*ileY*-dependent) and CBP-3xP-Cre (*proL*-dependent) were induced for 2h with 1mM IPTG. Fifteen µl of whole cell lysate were loaded on 4-20% PAGE-gels and stained with Coomassie blue. HcTnT is an *argU*-dependent T7-driven recombinant human cardiac troponin T expression construct (Hu et al.). CBP-3xi-Cre and CBP-3xP-Cre are T7-driven CBP-Cre-recombinase fusion genes containing either three AUA(3xi) codons or three CCC(3xP) codons near the N-terminus of the fusion protein. Cells with the RILP array (containing the *proL* tRNA gene), but not the RIL array, allow efficient expression of the recombinant CBP-3xP-Cre gene. The arrows indicate the position of the recombinant gene products. RIL: *argU, ileY* and *leuW* tRNA genes. RILP: *argU, ileY, leuW*, and *proL* tRNA genes.

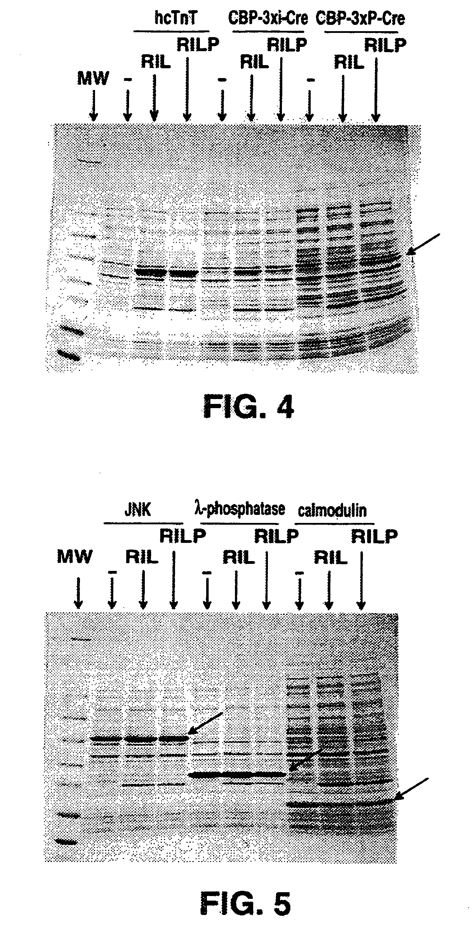

Figure 4 reveals that the RIL and RILP arrays do not significantly affect the level of expression of well-expressed proteins. The indicated host cells were transformed by T7-driven expression vectors for JNK (human c-jun N-terminal kinase), λ-phosphatase or calmodulin. Cultures were induced at mid-log growth for 2h with 1mM IPTG. Fifteen µl of induced cultures were denatured by boiling in SDS loading buffer, separated on a 4-20% PAGE gel and stained with Coomassie blue. The positions of the induced heterologous proteins are indicated by arrows. "-": BL21gold DE3. RIL: BL21gold DE3 with the *argU, ileY* and *leuW* tRNA gene array. RILP: BL21gold DE3 with the *argU, ileY, leuW* and *proL* tRNA gene array.
Figure 5 shows that high level expression of *Pfu*-polymerase depends on the presence of extra copies of both the *argU* and *ileY* genes. Cultures of BL21gold DE3 strains containing pACYC-based vectors expressing copies of the indicated *E.coli* tRNA genes and the T7-driven expression vectors for *Pfu*-polymerase, human cardiac troponin T and CBP/Cre-recombinase were induced for 2h with 1mM IPTG. Fifteen µl of whole cell lysate were loaded on 4-20% PAGE-gels and stained with Coomassie blue. High level of expression of human cardiac troponin T (hcTnT) in *E. coli* (expressed from a pET21b construct) is dependent on removal of two tandem AGA/AGG codons or rescue by extra copies of the *argU* tRNA gene. CBP-3xi-Cre is a CBP-tagged Cre-recombinase construct containing 3 extra AUA codons (coding for isoleucine) at the 5' - end of the recombinant gene. High level expression of this protein in *E*. *coli* is dependent on the presence of extra copies of *E*. *coli IleY* t-RNA gene (see Fig. 2). RG: pACYC with *argU* and *glyU* tRNA genes. RI: *argU* and *ileY* tRNA genes. IL: *ileY* and *leuW* tRNA genes. RIL: *argU, ileY*, and *leuW* tRNA genes.

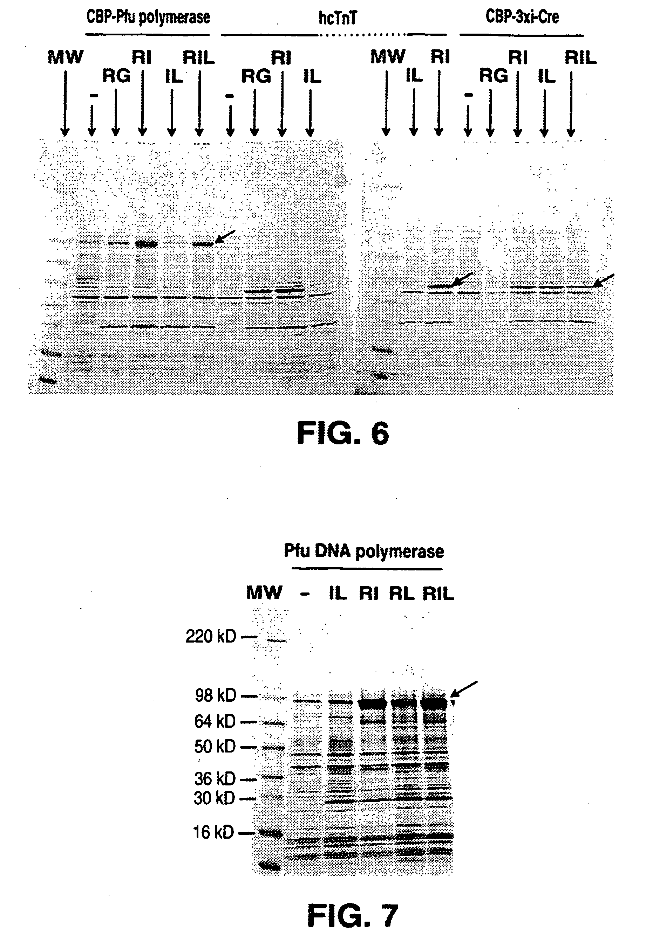

Figure 6 demonstrates that failure of the RG array to support efficient *Pfu* DNA-polymerase expression is not due to a negative effect of the *glyU* gene. The experiment displayed in Fig. 6 was repeated using an *argU*/*leuW* array (RL) instead of the *argU*/*glyU* array (RG), yielding the same result. Cultures of BL21goldDE3 strains containing pACYC-based vectors expressing copies of the indicated *E*. *coli* tRNA genes and the T7-driven expression vector for *Pfu* polymerase were induced for 2h with 1 mM IPTG. Fifteen µL of whole cell lysate were loaded on 4-20% PAGE gels and stained with Coomassie stain. RL: pACYC with *argU* and *leuW* tRNA genes. RI: *argU* and *ileY* tRNA genes. IL: *ileY* and *leuW* tRNA genes. RIL: *argU*, *ileY,* and *leuW* tRNA genes.
Figure 7 indicates that Pfu DNA polymerase I expression correlates with functional *argU* and *ileY* expression. *Pfu* DNA polymerase I was expressed in BL21goldDE3 strains either containing no tRNA expression vector (-), pACYC-RIL, or two different isolates of the RILP expression vector. Both RILP isolates displayed functional *argU* expression at the same level as observed with pACYC-RIL vector. However, RILP9 displays no functional *ileY* expression, and in RILP16 functional *ileY* expression is diminished when compared to RIL. Functional *ileY* expression was evaluated by rescue of the *ileY*-dependent production of CBP-3xi-Cre. Fifteen µl of the indicated cultures induced at mid-log growth for 2h with 1 mM IPTG were loaded on a 4-20% PAGE gel and the separated proteins were visualized by Coomassie blue staining.

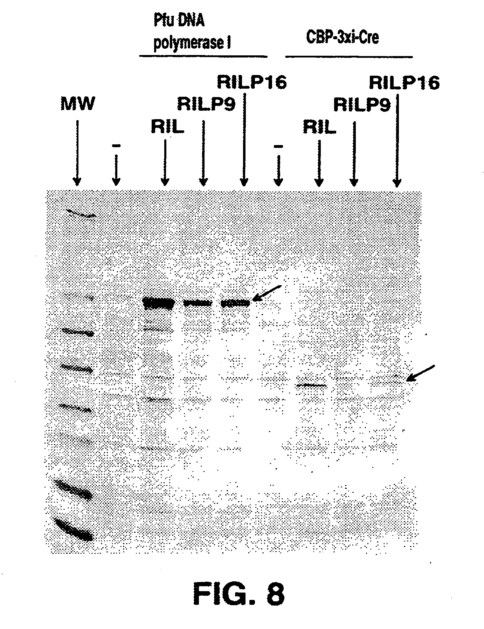

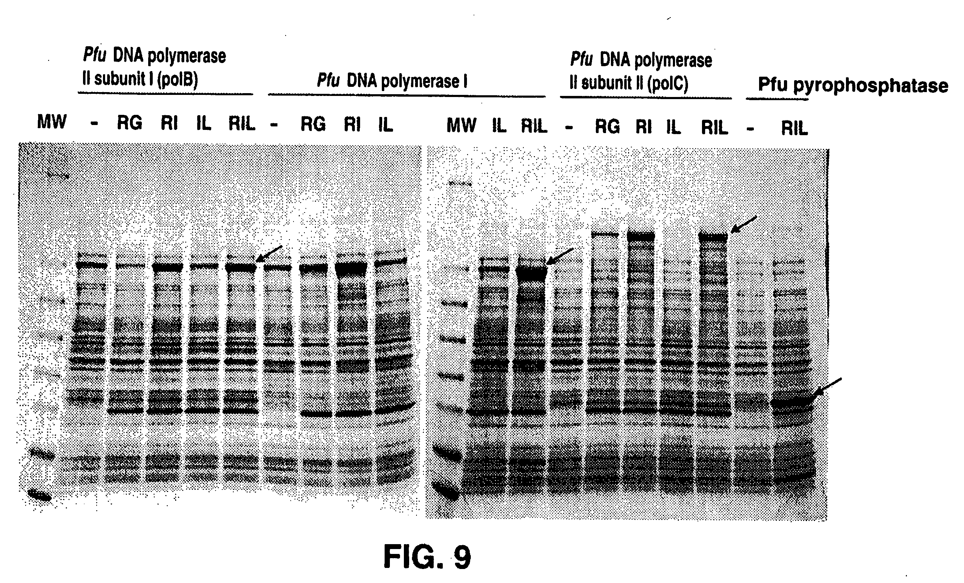

Figure 8 demonstrates that the expression of *Pfu*-polymerase II subunits I and II are dependent on functional co-expression of the *argU* and *ileY* genes. *Pfu* DNA polymerase I and the two sub-units of *Pfu*-DNA polymerase II were expressed in the indicated host strains. Fifteen µl of the cultures induced at mid-log growth for 2h with 1 mM IPTG were loaded on a 4-20% PAGE gel, and the separated proteins were visualized by Coomassie blue staining. "-": BL21gold DE3. RG: *argU* and *glyU* tRNA genes. RI: *argU* and *ileY* tRNA genes. IL: *ileY* and *leuW* tRNA genes. RIL: *argU, ileY*, and *leuW* tRNA genes.

### DETAILED DESCRIPTION OF THE INVENTION

There is a continuing need in the art for means to improve the efficiency of expression of heterologous proteins in *E. coli* and other host cells. This invention provides methods and cell lines that overcome the problem of poor codon usage, which limits the expression of certain heterologous proteins, and provides for higher levels of expression of such proteins. Poor codon usage is overcome in the present invention by identifying those codons rarely used in the host cell, yet required for expression of the protein of interest. The genes for the cognate tRNA species of the expression-limiting codons are then introduced into the host cell, thereby enabling a higher level of expression of the protein, *e*.*g*., at least two-fold higher than in the absence of the tRNA gene array.

As used herein, the term "host cell" refers to a cell used to express a protein of interest by incorporating a recombinant DNA molecule which encodes the protein. The host cell can be any type of prokaryotic or eukaryotic cell suitable for the expression of recombinant proteins. Frequently, the host cell will be a bacterium, a yeast cell, an insect cell, a plant cell or a mammalian cell.

This invention is directed toward the problem of rare codon usage in a host cell, which limits expression of a desired recombinant protein. "Codon usage" refers to the frequency with which a given codon appears in the coding regions of a gene. The codon usage of a host cell refers to the average codon usage for known genes which are endogenous to the host cell. A codon or its usage is "rare" if its frequency of use in the host cell is such that depletion of the corresponding tRNA species occurs during expression (particularly high level expression (see below)) of a heterologous protein of interest. A codon which is "rare" in a given host cell may be one which is normally not used by the host cell at all or which is used by the host cell in less than 1 % and even less than 0.5% of the host cell genes, or may be one which becomes limiting for the level of expression of a protein of interest. For example, several rare codons found in different organisms are presented in Tables 1-4.

A gene of interest which is introduced into a host cell is said to be "heterologous" if the frequency of codon usage for one or more codons in that gene is different from the frequency of codon usage for those codons in the host cell. For example, the gene of interest can have a different frequency of codon usage for one or several codons because the gene was isolated from another organism, which utilizes certain codons at a different frequency than the host cell. The organism from which a heterologous gene is obtained for use in the invention may be any organism. For example, the organism can be a prokaryote or eukaryote; it can be a bacterium, a yeast, a plant, an animal, or a mammal. A gene whose codon usage differs significantly from that of the host cell is said to have "biased codon usage." High level expression of a protein having biased codon usage can cause depletion of one or more tRNA species.

"High level expression" is the expression of a recombinant protein in a host cell in amounts higher than would be typical for an endogenous protein of the host cell. High level expression can result in the recombinant protein comprising from about 0.5%, 1%, 5%, 10% and even up to about 30% or more of total cell protein. High level expression is usually accomplished by introduction of the gene encoding the recombinant protein incorporated within an expression vector. An inducer can be added to the culture medium so as to activate the expression of the recombinant protein. For example, if the expression vector contains the promoter for T7 bacteriophage RNA polymerase, and the host cell chromosome contains the gene for T7 RNA polymerase under control of the *lac* repressor, then induction can be obtained by adding isopropylthio-β-galactoside (IPTG) to the medium. Suitable expression vectors are known in the art which, together with the appropriate host cell, will yield inducible, high level expression of a recombinant protein.

Any protein of interest can be employed in the invention, provided that it can be expressed from a recombinant DNA molecule in a suitable host cell. Preferably, the protein is one whose expression is limited by poor codon usage in the host cell. More preferably, a host cell is capable of high level expression of a protein of interest once the appropriate tRNA genes have been introduced into the host cell. Examples of proteins that are suitable for use with the invention are chicken histone H5 (Gerchman, S.E., Graziano, V., Ramakrishnan, V., "Expression of chicken linker histones in E. coli: Sources of problems and methods for overcoming some of the difficulties," Protein Express. Purif. 5:242-251 (1994), bovine placental lactogen (Kane, J.F., Violand, B.N., Curran, D.F., Staten, N.R., Duffin, K.L., Bogosian, G., "Novel in-frame two codon translational hop during synthesis of bovine placental lactogen in a recombinant strain of Escherichia coli," Nucleic Acids Res. 20:6707-6712 (1992), human tropoelastin (Martin, S.L., Vrhovski, B., Weiss, A.S., "Total synthesis and expression in Escherichia coli of a gene encoding human tropoelastin," Gene 154:159-166 (1995), human granulocyte-macrophage colony stimulating factor (Hua, Z., Wang. H., Chen, D., Chen, Y., Zhu, D., "Enhancement of expression of human ganulocyte-macrophage colony stimulating factor by argU gene product in Escherichia coli," Biochem. Mol. Biol. Int. 32:537-543 (1994)), human catechol-*O*-methyltransferase, as well as archebacterial proteins such as *Pyrococcus furiosus* DNA polymerases I and II.

### 1. Arrays of tRNA Genes Useful According to the Invention

The invention calls for introduction of an "array" of tRNA genes into the host cell. An array of tRNA genes consists of preferably 3 or more, e.g., 4, 5, 6, 7, 8, 9, or 10 tRNA genes which have been introduced into one or more vectors by cloning. As used herein, a "set" of tRNA genes contains only two tRNA genes; specific sets of tRNA genes useful according to the invention are described herein. A set, as used herein, does not consist of the pair of tRNA genes, *E*. *coli* genes *argU* and *ileX.*

According to one embodiment, an array of tRNA genes is introduced on a single vector. According to another embodiment, two or more vectors are introduced into the host cell, each containing either a single tRNA gene or an array of tRNA genes. In yet another embodiment, 2, 3, 4, 5, 6, 7, 8, 9, or 10 individual tRNA genes or one or more arrays of tRNA genes are inserted into the chromosome of the host cell, resulting in functional expression of the tRNA genes. In any embodiment of this invention, the tRNA genes introduced into the host cell may be different genes or multiple copies of identical tRNA genes. The vectors should be replication competent, suitable for transformation or transfection of the host cell, and should result in the functional expression of the tRNA molecules encoded by the genes.

The tRNA genes are functionally expressed if the host cell produces the corresponding functional tRNA molecules, which can be used in the translation of proteins in the host cell. The expression of the tRNA genes can be regulated by the endogenous promoter for each gene, or a different promoter or promoters can be added to control the transcription of any combination of the genes, including each gene individually or all of the genes simultaneously, under the control of a single promoter. The expression of two or more tRNA genes in the same host cell at the same time is referred to as "co-expression." Co-expression can be achieved, for example, by linking two or more tRNA genes to control by a single promoter.

The reported effects of rare codons generally refer to one codon occurring consecutively or in clusters. Reported experimental approaches have used either synonymous replacement of the codons or expression of a specific tRNA species along with a gene of interest. However, co-expression of an array of tRNA genes corresponding to rare codons according to the invention is contemplated to have a correspondingly greater effect on protein expression by permitting higher levels of expression of a selected protein than in cells lacking the tRNA gene array. Hence, expression of proteins containing multiple rare codons may be efficiently salvaged by simultaneous expression of their collective cognate tRNA genes.

The inventive approach to compensate for multiple rare codons is to generate a vector that co-expresses preferably three or more tRNA genes at the same time. Simultaneous expression of several tRNA genes from a vector also will generate a generic host for expression of genes from a variety of species with different codon biases. In a preferred embodiment, the vector is a low copy number vector. A low copy number vector is one having on average between about 1 and about 10 copies per cell. A medium copy number vector is one having on average between about 11 and about 50 copies per cell. A high copy number vector is one having on average between about 51 and about 200 copies per cell.

In one embodiment of the invention, the vector contains one or more transcription control elements in addition to the array of tRNA genes. A transcription control element is a nucleotide sequence that modifies, i.e., enhances or limits, transcription of the tRNA genes. A transcription control element can be, for example, a promoter or a terminator. A vector of the invention can also contain additional sequences, *e*.*g*., an origin of replication, a drug resistance gene, or a reporter gene, as required for its replication, function, or detection in the host cell, or as required for selection of transformed or transfected host cells.

A vector of the invention is selected for suitability of use with the chosen host strain. Regardless of the host cell type, the vector containing the tRNA gene array is preferably a low to medium copy number expression vector. The following are examples. Bacterial species that are suitable for use with the invention include *Escherichia coli* and *Bacillus subtilis.* Yeast species that are suitable for use with the invention include *Saccharomyces cerevisiae* and *Pichia pastoris.*

Specialized arrays of tRNA genes biased towards the codon usage of specific organisms are utilized according to the invention. Table 1 lists the codons that are used with a frequency of less than 1 % in *E. coli,* and compares the frequency of use in several other organisms. Frequently used codons (arbitrarily designated as codons with a frequency of more than 1.5 %) in heterologous genes constitute potential limitations to heterologous protein expression in *E. coli.* Using Table 1, or analogous tables prepared for other host cells, arrays of tRNA genes can be designed to meet the needs of a high level expression system for any organism. For example, one possible array for expression of human proteins in *E*. *coli* would include the cognate tRNA genes for codons AGG and AGA (Arg); CCC, CCU, and CCA (Pro); GGA and GGG (Gly); and UCC (Ser). Another example of an array that could be selected from Table 1 is the combination of arginine and proline codons (AGG and AGA (Arg); CCC (Pro)), which could be provided, for example, by the *E*. *coli* tRNA genes *argU* and *proL*. Yet another example of an array that could be selected from Table 1 is the combination of arginine, isoleucine, and leucine codons (AGG and AGA (Arg); AUA (Ile); and CUA (Leu)), provided, for example, by the *E*. *coli* tRNA genes *argU, ileY*, and *leuW*. By designing arrays of tRNA genes in this way, it is possible to tailor an expression host to meet the needs of any desired recombinant protein with a particular codon bias.

The host cell and a vector containing three or more tRNA genes may be made available as a kit. The vector of such a kit contains an array comprising preferably 3, 4, 5, 6, 7, 8, 9, or 10 tRNA genes tailored to the codon use frequency of, *e*.*g*., the proteins of a particular species and a particular host cell. In an alternative embodiment, the kit contains two or more vectors, each containing one or more tRNA genes or an array of tRNA genes. The user of the kit can choose particular tRNA genes to add to the array as needed for the production of a given heterologous protein with biased codon usage. For example, the vector of the kit can include a restriction endonuclease cleavage site, such that the user can cleave the vector, add two or more tRNA genes, and ligate the vector using a DNA ligase to form a new vector. The vector supplied with the kit is suitable for use with the host cell supplied with the kit. The kit also will include packaging materials for the vector and/or host cell.

Particularly useful sets of tRNA genes where only two tRNA genes are present in the set are as follows. In one such embodiment of this invention is a set containing two tRNA genes directed to the codons AGA or AGG (Arg) and AUA (Ile).

An array which targets the codons AGA or AGG (Arg)), CUA (Leu), AUA (Ile) and CCC (Pro) in *E*. *coli* is disclosed herein. The choice of these codons is based on their infrequent use in *E*. *coli* (see Table 1), and the availability of the cognate tRNA genes. To prepare this array of four tRNA genes which encode tRNAs rarely expressed in *E*. *coli, argU, ileY, leuW and proL* (recognizing the codons AGA/AGG, AUA, CUA and CCC, respectively) was introduced into a low copy number plasmid, pACYC-LIC (see Example 1). These four genes, which are rare in *E. coli,* were isolated from *E*. *coli* K12. Introduction of this plasmid into suitable protein expression hosts such as *E. coli* BL21DE3 allows high level of expression of proteins normally restricted by the presence of rare codons.

Further disclosed herein is a method of producing a protein of interest using a host cell and one or mre suitable vectors containing an array of tRNA genes. The gene for the protein of interest is introduced into the host cell or is present in the host cell. The gene can be introduced by any method known in the art. For example, the gene can be introduced by cotransforming or cotransfecting the host cell with an additional vector or nucleic acid construct which contains the gene and any nucleotide sequences necessary or desired to control transcription of the gene.

The method is used to produce the protein of interest, which is then isolated from the host cell and purified. Methods of protein purification are well known in the art and must be adapted to the requirements of each individual protein. The method applied to a given recombinant protein will depend on factors such as the size, charge, and hydrophobicity of the protein and whether the protein is secreted from the host cell, contained within the cytoplasm or within membranes, or precipitated in the form of inclusion bodies. See generally, Deutscher, M.P., "Guide to Protein Purification," Methods Enzymol., Academic Press, Vol. 182 (1990); Le, H.V., Trotta, P.P., "Purification of secreted recombinant proteins from Escherichia coli," Bioprocess Technol, 12:163-181 (1991); and Ladisch, M.R., Kolilmann, K.L., "Recombinant Human Insulin," Biotechnol. Prog, 8:469-478 (1992).

### 2. Host Cells Useful According to the Invention

### 2a. Bacterial Host Cells Useful According to the Invention

Suitable host cells for transfection with a set or array of tRNA genes and expressing the DNA encoding the desired polypeptide are the prokaryotic, yeast, or higher eukaryote cells. Suitable prokaryotes for this purpose include bacteria such as archaebacteria and eubacteria. Preferred bacteria are eubacteria, such as Gram-negative or Gram-positive organisms, for example, *Enterobacteriaceae* such as *Escherichia, e*.*g*., *E. coli, Enterobacter, Erwinia, Klebsiella, Proteus, Salmonella, e.g., Salmonella typhimurium, Serratia*, e.g., *Serratia marcescans*, and *Shigella; Bacilli* such as *B*. *subtilis* and *B. licheniformis* (*e.g., B. licheniformis* 41P disclosed in DD 266,710 published Apr. 12, 1989); *Pseudomonas* such as *P. aeruginosa, Streptomyces; Azotobacter; Rhizobia; Vitreoscilla; and Paracoccus.* Suitable *E. coli* hosts include *E*. *coli* W3110 (ATCC 27,325), *E. coli* 294 (ATCC 31,446), *E. coli* B, and *E. coli* X1776 (ATCC 31,537). These examples are illustrative rather than limiting.

Mutant cells of any of the above-mentioned bacteria may also be employed. It is, of course, necessary to select the appropriate bacteria taking into consideration replicability of the replicon in the cells of a bacterium. For example, *E. coli, Serratia,* or *Salmonella* species can be suitably used as the host when well known plasmids such as Col El, pACYC and RKZ-based vectors are used to supply the replicon. *E. coli* strain W3110 is a preferred host or parent host because it is a common host strain for recombinant DNA product fermentations. Preferably, the host cell should secrete minimal amounts of proteolytic enzymes or should be protease deficient. Cells which are lacking or deficient in Lon and OmpT proteases are preferred. For example, strain W3110 may be modified to effect a genetic mutation in the genes encoding proteins, with examples of such hosts including *E. coli* W3110 strain 27C7. The complete genotype of 27C7 is tonA.DELTA.ptr3 phoA.DELTA.E15.DELTA.(argF-lac) 169 ompT.DELTA. degP41kan.sup.r. Strain 27C7 was deposited on Oct. 30, 1991 in the American Type Culture Collection as ATCC No. 55,244. Alternatively, the strain of *E*. *coli* having mutant periplasmic protease disclosed in U.S. Pat. No. 4,946,783 issued Aug. 7, 1990 may be employed. Alternatively, *in vitro* methods of cloning, *e*.*g*., PCR or other nucleic acid polymerase reactions, are suitable. *E. coli* EL21 also is useful according to the invention (Studier et al., 1986, J. Mol. Biol., 189:113; Studier et al., 1990, Methods Enzy. 185:60). Another preferred attribute of the host cell is the Hte (high transformation efficiency) phenotype, which increases the the transformation efficiency of large and ligated DNA. RecA positive strains allow faster growth and yield high levels of protein expression. Endonuclease I (*e*.*g*., EndA 1) deficient strains yield miniprep DNA that is less prone to degradation.

For example, strain W3110 may be modified to effect a genetic mutation in the genes encoding proteins endogenous to the host, with examples of such hosts including *E. coli* W3110 strain 1A2, which has the complete genotype tonA.DELTA.; *E. coli* W3110 strain 9E4, which has the complete genotype tonA.DELTA. ptr3; *E. coli* W3110 strain 27C7 (ATCC 55,244), which has the complete genotype tonA.DELTA. ptr3 phoA.DELTA.E15 (argF-lac)169 ompT.DELTA. degP41kan.sup.r ; *E. coli* W3110 strain 37D6, which has the complete genotype tonA.DELTA. ptr3 phoA.DELTA.E15 .DELTA.(argF-lac)169 ompT.DELTA. degP41kan.sup.r rbs7.DELTA. ilvG; *E. coli* W3110 strain 40B4, which is strain 37D6 with a non-kanamycin resistant degP deletion mutation; and an *E. coli* strain having mutant periplasmic protease disclosed in U.S. Pat. No. 4,946,783 issued Aug. 7, 1990.

### 2b. Fungi, Plant and Insect Host Cells Useful According to the Invention

In addition to prokaryotes, eukaryotic microbes such as filamentous fungi or yeast are suitable cloning or expression hosts for set or arrays of tRNA genes and polypeptide-encoding vectors. *Saccharomyces cerevisiae*, or common baker's yeast, is the most commonly used among lower eukaryotic host microorganisms. However, a number of other genera, species, and strains are commonly available and useful herein, such as *Schizosaccharomyces pombe* (Beach and Nurse, Nature, 290: 140 (1981); EP 139,383 published May 2, 1985); *Kluyveromyces hosts* (U.S. Pat. No. 4,943,529; Fleer et al., supra) such as, e.g., *K. lactis* (MW98-8C, CBS683, CBS4574; Louvencourt et al., *J. Bacteriol*., 737 (1983)), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., supra), *K. thermotolerans*, and *K. marxianus; yarrowia* (EP 402,226); *Pichia pastoris* (EP 183,070; Sreekrishna et al., J. Basic Microbiol., 28: 265-278 (1988)); *Candida; Trichoderma reesia* (EP 244,234); *Neurospora crassa* (Case et al. , Proc. Natl. Acad. Sci. USA, 76: 5259-5263 (1979)); *Schwanniomyces* such as *Schwanniomyces occidentalis* (EP 394,538 published Oct. 31, 1990); and filamentous fungi such as. e.g., *Neurospora, Penicillium, Tolypocladium* (WO 91/00357 published Jan. 10, 1991), and *Aspergillus* hosts such as *A. nidulans* (Ballance et al., Biochem. Biophys, Res. Commun., 112: 284-289 (1983); Tilburn et al., Gene, 26: 205-221 (1983); Yelton et al., Proc. Natl. Acad. Sci. USA, 81: 1470-1474 (1984)) and *A. niger* (Kelly and Hynes, EMBO J., 4: 475-479 (1985)).

Suitable host cells according to the invention and appropriate for the expression of the DNA encoding the desired polypeptide may also be derived from multicellular organisms. Such host cells are capable of complex processing and glycosylation activities. In principle, any higher eukaryotic cell culture is suitable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. See, e.g., Luckow et al., Bio/Technology, 6: 47-55 (1988); Miller et al., in Genetic Engineering, Setlow, J. K. et al., eds., Vol. 8 (Plenum Publishing, 1986), pp. 277-279; and Maeda et al., Nature, 315: 592-594 (1985). A variety of viral strains for transfection are publicly available, *e.g*., the L-1 variant of *Autographa californica* NPV and the Bm-5 strain of *Bombyx mori* NPV, and such viruses may be used as the vector herein according to the present invention, particularly for transfection of *Spodoptera frugiperda* cells.

Plant cell cultures of cotton, corn, potato, soybean, petunia, tomato, and tobacco can be utilized as hosts. Typically, plant cells are transfected by incubation with certain strains of the bacterium Agrobacterium tumefaciens, which has been previously manipulated to contain the DNA encoding the desired polypeptide. During incubation of the plant cell culture with *A. tumefaciens*, the DNA encoding the desired polypeptide is transferred to the plant cell host such that it is transfected, and will, under appropriate conditions, express the DNA encoding the desired polypeptide. In addition, regulatory and signal sequences compatible with plant cells are available, such as the nopaline synthase promoter and polyadenylation signal sequences (Depicker et al., J. Mol. Appl. Gen., 1:561, (1982)). In addition, DNA segments isolated from the upstream region of the T-DNA 780 gene are capable of activating or increasing transcription levels of plant-expressible genes in recombinant DNA-containing plant tissue. EP 321,196 published Jun. 21, 1989.

### 2c. Mammalian Host Cells Useful According to the Invention

Examples of useful mammalian host cell lines are monkey kidney Cv1 line transformed by SV40 (COS-7, ATCC CRL 1651); human embryonic kidney line (293 or 293 cells subcloned for growth in suspension culture, Graham et al., J. Gen Virol., 36: 59 (1977)); baby hamster kidney cells (BHK, ATCC CCL 10); Chinese hamster ovary cells/-DHFR (CHO, Urlaub and Chasin, Proc.Natl. Acad. Sci. USA, 77: 4216 (1980)); mouse sertoli cells (TM4, Mather, Biol. Reprod., 23: 243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W 138, ATCC CCL 75); human liver cells (Hep G2, HB 8065); mouse mammary tumor (MMT 060562, ATCC CCL51); TRI cells (Mather et al., Annals N. Y. Acad. Sci., 383: 44-68 (1982)); MRC 5 cells; FS4 cells; and a human hepatoma line (Hep G2).

Host cells are transfected and preferably transformed with the above-described set or array of tRNA genes and expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO4 and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in section 1.82 of Sambrook et al., Molecular Cloning: A Laboratory Manual (New York: Cold Spring Harbor Laboratory Press, 1989), or electroporation is generally used for prokaryotes or other cells that contain substantial cell-wall barriers. Infection with *Agrobacterium tumefaciens* is used for transformation of certain plant cells, as described by Shaw et al., Gene, 23: 315 (1983) and WO 89/05859 published Jun. 29, 1989. In addition, plants may be transformed using ultrasound treatment as described in WO 91/00358 published Jan. 10, 1991.

For mammalian cells without such cell walls, the calcium phosphate precipitation method of Graham and van der Eb, Virology, 52: 456-457 (1978) is preferred. General aspects of mammalian cell host system transformations have been described by Axel in U.S. Pat. No. 4,399,216 issued Aug. 16, 1983. Transformations into yeast are typically carried out according to the method of Van Solingen et al., J. Bact., 130: 946 (1977) or Hsiao et al., Proc. Natl. Acad. Sci. USA, 76: 3829 (1979). However, other methods for introducing DNA into cells, such as by nuclear microinjection, electroporation, bacterial protoplast fusion with intact cells, or polycations, *e*.*g*., polybrene, polyornithine, etc., may also be used. For various techniques for transforming mammalian cells, see Keown et al., Methods in Enzymology (1989), Keown et al., Methods in Enzymology (1990) Vol. 185, pp. 527-537, and Mansour et al., Nature, 336: 348-352 (1988).

If prokaryotic cells are used to produce the polypeptide of interest in accordance with the method of this invention, they are cultured in suitable media in which the promoter can be constitutively or artificially induced as described generally, *e*.*g*., in Sambrook et al., supra, 1989.

Any necessary supplements besides carbon, nitrogen, and inorganic phosphate sources may also be included at appropriate concentrations introduced alone or as a mixture with another supplement or medium such as a complex nitrogen source. The pH of the medium may be any pH from about 5 to 9, depending mainly on the host organism.

If mammalian host cells are used to produce the polypeptide of this invention, they may be cultured in a variety of media. Commercially available media such as Ham's F10 (Sigma), Minimal Essential Medium ((MEM), Sigma), RPMI-1640 (Sigma), and Dulbecco's Modified Eagle's Medium (DMEM), Sigma) are suitable for culturing the host cells. In addition, any of the media described in Ham and Wallace, Meth. Enz., 58: 44 (1979), Barnes and Sato, Anal. Biochem., 102: 255 (1980), U.S. Pat. Nos. 4,767,704; 4,657,866; 4,927,762; or 4,560,655; WO 90/03430; WO 87/00195; U.S. Pat. Re. No. 30,985; or U.S. Pat. No. 5,122,469, the disclosures of all of which are incorporated herein by reference, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin TM. drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, or can be modified as needed to obtain optimal expression, and will be apparent to the ordinarily skilled artisan. In general, principles, protocols, and practical techniques for maximizing the productivity of *in vitro* mammalian cell cultures can be found in Mammalian Cell Biotechnology: A Practical Approach, M. Butler, ed., (IRL Press at Oxford University Press, Oxford, 1991).

### 3. Purification of a Polypeptide of Interest From a Host Cell

It is often preferred to purify the polypeptide of interest from recombinant cell proteins or polypeptides to obtain preparations that are substantially homogeneous as to the polypeptide of interest. In one embodiment, as a first step, the culture medium or lysate is centrifuged to remove particulate cell debris. The membrane and soluble protein fractions may then be separated if necessary. The polypeptide may then be purified from the soluble protein fraction and from the membrane fraction of the culture lysate, depending on whether the polypeptide is membrane bound, is soluble, or is present in an aggregated form. The polypeptide thereafter is solubilized and then subsequently refolded if necessary using an appropriate buffer.

Insoluble, non-native polypeptide is isolated from the prokaryotic host cells in a suitable isolation buffer by any appropriate technique, *e*.*g*., one involving exposing the cells to a buffer of suitable ionic strength to solubilize most host proteins, but in which aggregated polypeptide is substantially insoluble, disrupting the cells so as to release the inclusion bodies and make them available for recovery by, for example, centrifugation. This technique is well known, and is described, for example, in U.S. Pat. No. 4,511,503.

Briefly, the cells are suspended in the buffer (typically at pH 5 to 9, preferably about 6 to 8, using an ionic strength of about 0.01 to 2M, preferably 0.1 to 0.2M). Any suitable salt, including sodium chloride, is useful to maintain a sufficient ionic strength value. The cells, while suspended in this buffer, are then disrupted by lysis using techniques commonly employed such as mechanical methods (*e*.*g*., a Manton-Gaulin press, a French press, or a sonic oscillator), or by chemical or enzymatic methods.

Examples of chemical or enzymatic methods of cell disruption include spheroplasting, which entails the use of lysozyme to lyse the bacterial wall (Neu et al., Biochem. Biophys. Res. Comm., 17: 215 (1964)), and osmotic shock, which involves treatment of viable cells with a solution of high tonicity and with a cold-water wash of low tonicity to release the polypeptides (Neu et al., J. Biol. Chem., 240: 3685-3692 (1965)). A third method, described in U.S. Pat. No. 4,680,262 issued Jul. 14, 1987, involves contacting the transformed bacterial cells with an effective amount of a lower alkanol having 2 to 4 carbon atoms for a time and at a temperature sufficient to kill and lyse the cells.

After the cells are disrupted, the suspension is typically centrifuged to pellet the inclusion bodies. In one embodiment, this step is carried out at about 500 to 15,000 x g, preferably about 12,000 x g, in a standard centrifuge for a sufficient time that depends on volume and centrifuge design, usually about 10 minutes to 0.5 hours. The resulting pellet contains substantially all of the insoluble polypeptide fraction, but if the cell disruption process is not complete, it may also contain intact cells or broken cell fragments. Completeness of cell disruption can be assayed by resuspending the pellet in a small amount of the same buffer solution and examining the suspension with a phase contrast microscope. The presence of broken cell fragments or whole cells indicates that additional disruption is necessary to remove the fragments or cells and the associated non-refractile polypeptides. After such further disruption, if required, the suspension is again centrifuged and the pellet recovered, resuspended, and analyzed. The process is repeated until visual examination reveals the absence of broken cell fragments in the pelleted material or until further treatment fails to reduce the size of the resulting pellet.

In an alternative embodiment, the polypeptide of interest, preferably exogenous, is isolated by solubilization in a suitable buffer. This procedure can be *in situ* solubilization involving direct addition of reagents to the fermentation vessel after the polypeptide has been produced recombinantly, thereby avoiding extra steps of harvesting, homogenization, and centrifugation to obtain the polypeptide. The remaining particulates can be removed by centrifugation or filtration, or combinations thereof. Alternatively, and more preferably, one may use a multiple-phase isolation/extraction system for purifying polypeptides from the remaining particulates.

The above disclosure generally describes the present invention. A more complete understanding can be obtained by reference to the following specific examples, which are provided herein for purposes of illustration only and are not intended to limit the scope of the invention.

### EXAMPLE 1

### Construction of expression vectors containing E. coli tRNA gene sets or arrays.

Expression vectors for tRNA gene sets or arrays, with the exception of pACYC-RG, were constructed by inserting PCR-amplified tRNA gene sets or arrays containing *Spe*I and *Xho*I sites at their 5' and 3' end between the *Spe*I and *Xho*I sites of the pACYC 1 84 derivative pACYC-LIC. pACYC-LIC contains a multiple cloning site including LIC-sites replacing most of the *tet* gene of pACYC184. The *tet* promoter is retained and drives expression of inserts within the polylinker.

The RG-set was cloned into pACYC-LIC by ligation-independent cloning. The sets were generated by first amplifying the individual tRNA genes with primers including recognition sites for restriction enzymes, ligating the cut tRNA fragments to each other and then amplifying the desired sets or arrays with flanking primers using the ligation reaction as the template. The RILP array was generated by first ligating the *proL* gene fragment to the RIL array and then amplifying the whole array by using the flanking primer as described for the other arrays. For an overview of the constructs see Figure 1. Hereinafter, R will refer to the *argU* tRNA gene, I to the *ileY* tRNA gene, L to the *leuW* tRNA gene, P to the tRNA gene and G to the *glyU* tRNA gene when used in the context of tRNA gene sets or arrays or the derived vectors.

The individual tRNA genes were isolated by PCR using *E.coli* K12 DNA as a template. ArgU was amplified using the primer GAC ACT AGT AAT CAG ACG CGG TCG TTC AC (SEQ ID NO:1; for RI, RIL and RILP; *Spe*I site underlined) or GAC GAC GAC AAG AAT CAG ACG CGG TCG TTC AC (SEQ ID NO:2; for RG; LIC site underlined) as forward primer and CTG CCA TGG TGG AGG ATA TAA AGA AGG CG (SEQ ID NO:3; *Nco*I site underlined) as the reverse primer. The primers anneal at bp 8041 (forward) and bp 8260 (reverse) in the Genbank file Accession Number AE000159. The amplified fragment is 220 bp long and contains 104 bp 5' and 38 bp 3' to the *argU* tRNA gene. Extensions containing recognition sites for *Spel* (forward primer) and *Nco*I restriction endonucleases were added for construction purposes. The *ileY* tRNA gene was amplified using the primer CAG CCA TGG CCT TGA AAT GGC GTT AGT CA (SEQ ID NO:4; for RI and RIL; *Nco*l site underlined) or GAC ACT AGT CCT TGA AAT GGC GTT AGT CA (SEQ ID NO:5; for IL; *Spel* site underlined) as forward primer and CAG TCT AGA TCA TCA TGT TTA TTG CGT GG (SEQ ID NO:6; for IL and RIL; *Xba*I site underlined) or GAC CTC GAG TCA TCA TGT TTA TTG CGT GG (SEQ ID NO:7; for RI; *Xho*I site underlined) as the reverse primer. The primers anneal at bp 7741 and bp 7950 in the Genbank file Accession Number AE000350. The amplified fragment is 210 bp long and contains 92 bp 5' and 54 bp 3' to the *IleY* tRNA gene. Extensions containing recognition sites for *Nco*l or *Spel* (forward primer) and *Xba*l or *Xho*l (reverse primer) restriction endonucleases were added for construction purposes. The *leuW* tRNA gene was amplified using the primer CAG TCT AGA GAA TCC CGT CGT AGC CAC CA (SEQ ID NO:8; *Xba*l site underlined) as forward primer and GAC CTC GAG GGC ATC CGA TCA ACG CTT TCT (SEQ ID NO:9; *Xhol* site underlined) as the reverse primer. The primers anneal at bp 241 (forward) and bp 378 (reverse) in the Genbank file Accession Number J01713. The amplified fragment is 138 bp long and contains 29 bp 5' and 33 bp 3' to the *LeuW* tRNA gene. Extensions containing recognition sites for *Xba*I (forward primer) and *Xho*l restriction endonucleases were added for construction purposes. The *proL* tRNA gene was amplified using the primer GAC GTC GAC GTG CTG ACA GAC GAG AAG CG (SEQ ID NO:10; *Sal*I site underlined) as forward primer and GAC CTC GAG GGT GTG GTC TGG ACG TTC TG (SEQ ID NO:11) as reverse primer. The amplified product is 310 bp long and contains 110 bp 5' and 117 bp 3' to the tRNA gene. The *Sal*I and *Xho*I sites were included for construction purposes. The *glyU* tRNA gene was amplified using the primer CTG CCA TGG GGC ACT TGC TAA GGA GAG CG (SEQ ID NO:12; *Nco*I site underlined) as forward primer and GGA ACA AGA GGG CGT GTT TTC CTG GGT TGT TAC (SEQ ID NO:13; LIC site underlined) as the reverse primer. The amplified fragment is 209 bp long and contains 49 bp 5' and 86 bp 3' to the tRNA gene. All PCR reactions were carried out for 30 cycles of 95°C, 1 min, 55°C, 1 min and 72°C for 1 min using cloned *Pfu*-polymerase supplemented with PEF, except for *proL*, which was amplified using Taq Plus precision (Stratagene).

For amplification of the sets or arrays the respective PCR products were isolated from agarose gels and digested with the appropriate restriction enzymes. The restriction digested PCR fragments were then ligated to each other for 4 h at 16°C using T4 DNA ligase. One µl of this ligation was used as a template in a PCR reaction using the forward primer of the first tRNA gene and reverse primer of the last tRNA gene of the desired set or array. The conditions for the PCR reactions were the same as described above except that only 25 cycles were performed. The reaction product of the anticipated size for the correct product was isolated from an agarose gel, digested with *Spel* and *Xho*I and ligated into *Spe*I and *Xho*I-digested pACYCI84-LIC. The map of the resulting plasmids is shown in Fig. 1.

### EXAMPLE 2

### Test of Functional Expression of the argU, ileY, and proL tRNA Genes.

The tRNA genes in the vector were arranged in an operon-like structure driven by the promoter of the tetracycline resistance gene (Fig 1). Some of the fragments may have contained their endogenous promoter. The fragment containing the *argU* gene contained its own promoter (Saxena and Walker, "Expression of argu, the Escherichia coli gene coding for a rare arginine tRNA," J. Bacterio/. 174:1956-64 (1992)), but lacked the rho-dependent transcriptional terminators (Garcia, G.M., Mar, P.K., Mullin, D.A., Walker, J.R., Prather, N.E., "The E. coli dnaY gene encodes an arginine transfer RNA," Cell 45:453-459 (1986)). The affected sequence was not part of the mature tRNA. The mutations may be significant for expression, however, since they disrupt a dyad symmetry element that previously has been demonstrated to suppress transcription of the *argU* promoter (Saxena and Walker, 1992, supra). The promoter regions for the *ileY* and *proL* genes are not defined. However, based on their natural arrangement as isolated tRNA genes within the *E. coli* genome the two fragments utilized can be expected to contain their own promoters. In its natural arrangement the *leuW* gene is part of a tRNA operon, and the amplified fragment may therefore lack an individual promoter (Nakajima. N., Ozeki, H., Shimura, Y., "Organization and structure of an E. coli tRNA operon containing seven tRNA genes," Cell 23:239-49 (1981)).

Since genes affected by AUA (ile), CGA (leu) or CCC (pro) were not available, tester constructs were generated based on the observation (Rosenberg, 1993, *supra*) that rare codons affect translation in *E. coli* most when present in a consecutive arrangement at the N-terminus of the protein. Three consecutive leucine (CUA), isoleucine (AUA), or proline (CCC) codons were introduced at the 5' end of the recombinant CBP/Cre fusion gene. Upon IPTG induction, unmodified CBP/Cre fusion protein was expressed at approximately 30% of the total protein, most, if not all of it being soluble. As can be seen in Figure 2, introduction of the rare leucine codon CUA did not affect expression of the recombinant gene. The functional expression of the *leuW* gene could therefore not be assessed. In contrast, introduction of the isoleucine codon strongly reduced expression of the recombinant protein. Expression could be rescued by expression of sets or arrays containing the *ileY* gene, but not by the RG (*argU and* glyU) set, which lacks the *ileY* gene. Thus, the *ileY* gene in the RIL array is functional and specifically rescues expression of genes affected by the rare isoleucine codon AUA.

The same strategy was applied to test for functional expression of the *proL* gene in the RILP array. As can be seen in Figure 3, the presence of the RILP array, but not the RIL array, rescued expression of a tester construct containing the cognate CCC codon. Therefore, the *proL* gene in the RILP array is functional. However, although the *argU* gene in the RILP array performs at the same level as observed in the RIL array, functional expression of the *ileY* gene in the RILP array was diminished when compared to the RIL array. Five independent isolates of the pACYC-RILP construct were tested and the same effect was observed in all constructs, albeit to a different extent (ranging from undetectable expression to detectable, but diminished). Sequencing the RILP-tRNA gene arrays revealed that in isolates failing to show *ileY* activity, the *ileY* gene contains a point mutation (A -- > T at nt 50 of the mature tRNA) that prevents proper folding of the tRNA and thus is likely to inactivate it. However, in the RILP isolates displaying diminished activity, the sequences of the *argU, ileY,* and *leuW* tRNA genes were indistinguishable from the RIL array. This observation suggests a potential incompatibility of simultaneous ectopic expression of the *proL* and *ileY* tRNA genes, possibly due to either interference of the *proL* gene with *ileY* transcription and/or processing or due to an attenuation of the host cells.

### EXAMPLE 3

### Effect of RIL and RILP Arrays on Expression of Well-Expressed Genes.

In order to test for potential deleterious effects of the RIL and RILP arrays, the expression of four well-expressing T7-driven recombinant genes were compared in BL21goldDE3 cells and their pACYC-RIL and pACYC-RILP containing derivatives. As shown in Fig. 4, no differences were observed between BL21goldDE3 cells and their tRNA-supplemented derivatives in the induced expression level of λ-phosphatase or JNK. However, a loss of expression was observed for calmodulin. The differences between the lines for calmodulin expression were significantly smaller when the induction time was extended, and great variations in induction efficiencies were observed for calmodulin but not for any of the other tested constructs. The reason for the suppression of calmodulin expression is unclear. For chemically competent pACYC-RIL cells, transformation efficiencies of 1 x 10⁸ /µg of pUC18 could be achieved, which was the expected efficiency for a BL21goldDE3 derived cell line. Aside from the induction of calmodulin, no negative effects of the pACYC-based tRNA expression arrays on host cell performance have been detected.

### EXAMPLE 4

### Enhancement of Expression of Archebacterial Genes.

In order to find further examples of genes aided in their bacterial expression by ectopic tRNA expression, six recombinant *Pyrococcus furiosus* genes were tested that are only expressed poorly in *E*. *coli.* The choice of archaebacterial genes was based on their strong bias for rare *E*. *coli* codons, especially AGG/AGA and AUA (Table 1; in fact, 95 % of the arginine codons are AGG or AGA). Of six tested constructs, four were rescued by co-expression of the pACYC-RIL construct. The enhanced expression of *Pfu*-polymerase I, *Pfu* polymerase II subunit I, *Pfu* polymerase II subunit II, and *Pfu* pyrophosphatase is shown in Figs. 5 and 8.

### EXAMPLE 5

### Enhancement of Gene Expression by Simultaneous Co-Expression of Several tRNA Genes.

Judging by the codon usage of *Pyrococcus furiosus,* genes from this organism are expected to be affected by AGG/AGA (arginine) and AUA (isoleucine) codons. The sequence of *Pfu* DNA polymerase I contains several pairs of rare arginine and isoleucine codons. In order to test the effect of the simultaneous presence of extra copies of the *argU and ileY* genes on expression of *Pfu*-polymerase, IPTG-induced expression level of this gene was compared in strains containing extra copies of different combinations of tRNA genes. As shown in Fig. 5, expression of *Pfu* DNA polymerase I is enhanced by the presence of the *argU* gene in the RG set (*argU* and *glyU*) but to a significantly smaller degree than is achieved by a set or array containing both the *argU* and *ileY* genes (RI and RIL). Thus, simultaneous expression of *argU* and *ileY* yielded about 5-fold higher expression of *Pfu* DNA polymerase I than expression on either gene alone. The set containing only the *ileY* gene but not the *argU* gene (IL) failed to enhance expression of *Pfu* polymerase when compared to BL21goldDE3 cells. However, the *argU* gene in the RG set and the *ileY* gene in the IL set were sufficient to rescue expression of constructs only affected by AGG/AGA codons (hcTnT) or AUA codons (CBP-3xi-Cre) to the same level as was observed with the RI and RIL. The lack of efficient *Pfu* DNA polymerase production in host cells containing the RG set was not due to potentially negative effects of the *glyU* tRNA, because the same effect was observed in cells carrying the RL set (Fig. 6).

As described herein, the expression of the *ileY* gene is compromised in different isolates of the RILP array. Two isolates were used (RILP9, lacking detectable functional *ile Y* and RILP 16, displaying diminished *ile Y* function when compared to RIL) to demonstrate the dose-dependence of *Pfu* DNA polymerase expression on *ileY* expression. As shown in Fig. 7 the expression of *Pfu* DNA polymerase in the RIL and RILP strains correlates with the functional expression of *ileY.* Thus, efficient expression of *Pfu* DNA polymerase in *E.coli* is dependent on the simultaneous presence of extra copies of the *argU* as well as the *ileY* gene.

To test whether the improved expression of the target gene by simultaneous expression of several different tRNA genes is restricted to *Pfu*-polymerase 1, two additional proteins, *Pfu* polymerase II subunit I and subunit II were tested. Judged by their sequence, both genes should be similarly affected. In fact, expression of both genes was enhanced about 5-fold by coexpression of the *argU and glyU* tRNA genes (Fig. 8), similar to the observation with *Pfu* DNA polymerase 1. This suggests that the requirement for extra copies of two or more different tRNA genes for efficient gene expression in *E. coli* may be a frequent phenomenon for genes derived from organisms possessing a distinct codon usage from that of *E*. *coli.*

### OTHER EMBODIMENTS

Other embodiments of the invention are within the following claims.
<110> Stratagene
<120> Methods and Compositions for High Level Expression of a Heterologous Protein with Poor Codon Usage
<130> Stratagene tRNA
<140>
   <141>
<160> 13
<170> PatentIn Ver. 2.1
<210> 1
   <211> 29
   <212> DNA
   <213> Artificial Sequence
<220>
   <223> Description of Artificial Sequence: PCR primer for E. coli argU gene
<400> 1
   gacactagta atcagacgcg gtcgttcac
   29
<210> 2
   <211> 32
   <212> DNA
   <213> Artificial Sequence
<220>
   <223> Description of Artificial Sequence: PCR primer for E. coli argU gene
<400> 2
   gacgacgaca agaatcagac gcggtcgttc ac
   32
<210> 3
   <211> 29
   <212> DNA
   <213> Artificial Sequence
<220>
   <223> Description of Artificial Sequence: PCR primer for E. coli argU gene
<400> 3
   ctgccatggt ggaggatata aagaaggcg
   29
<210> 4
   <211> 29
   <212> DNA
   <213> Artificial Sequence
<220>
   <223> Description of Artificial Sequence: PCR primer for E. coli ileY gene
<400> 4
   cagccatggc cttgaaatgg cgttagtca
   29
<210> 5
   <211> 29
   <212> DNA
   <213> Artificial Sequence
<220>
   <223> Description of Artificial Sequence: PCR primer for E. coli ileY gene
<400> 5
   gacactagtc cttgaaatgg cgttagtca
   29
<210> 6
   <211> 29
   <212> DNA
   <213> Artificial Sequence
<220>
   <223> Description of Artificial Sequence: PCR primer for E. coli ileY gene
<400> 6
   cagtctagat catcatgttt attgcgtgg
<210> 7
   <211> 29
   <212> DNA
   <213> Artificial Sequence
<220>
   <223> Description of Artificial Sequence: PCR primer for E. coli ileY gene
<400> 7
   gacctcgagt catcatgttt attgcgtgg
   29
<210> 8
   <211> 29
   <212> DNA
   <213> Artificial Sequence
<220>
   <223> Description of Artificial Sequence: PCR primer for E. coli leuW gene
<400> 8
   cagtctagag aatcccgtcg tagccacca
   29
<210> 9
   <211> 30
   <212> DNA
   <213> Artificial Sequence
<220>
   <223> Description of Artificial Sequence: PCR primer for E. coli leuW gene
<400> 9
   gacctcgagg gcatccgatc aacgctttct
   30
<210> 10
   <211> 29
   <212> DNA
<220>
   <223> Description of Artificial Sequence: PCR primer for E. coli proL gene
<400> 10
   gacgtcgacg tgctgacaga cgagaagcg
   29
<210> 11
   <211> 29
   <212> DNA
   <213> Artificial Sequence
<220>
   <223> Description of Artificial Sequence: PCR primer for E. coli proL gene
<400> 11
   gacctcgagg gtgtggtctg gacgttctg
   29
<210> 12
   <211> 29
   <212> DNA
   <213> Artificial Sequence
<220>
   <223> Description of Artificial Sequence: PCR primer for E. coli glyU gene
<400> 12
   ctgccatggg gcacttgcta aggagagcg
   29
<210> 13
   <211> 33
   <212> DNA
   <213> Artificial Sequence
<220>
   <223> Description of Artificial Sequence: PCR primer for E. coli glyU gene

## Claims

1. A pACYC-LIC nucleic acid vector comprising sequences encoding *E. coli argU, ileY* and *leuW* tRNAs.

2. The vector of claim 1, wherein said sequences encoding said *E. coli* tRNAs are present in the order argU, ileY, leuW.

3. The vector according to claim 1 or claim 2, wherein said vector comprises a *tet* promoter.

4. The vector of claim 3, wherein said *tet* promoter drives expression of the sequence encoding said tRNAs.

5. The vector according to any of claims 1-4, wherein said sequence encoding *E.coli argU* tRNA comprises the sequence between base pairs 8041 and 8260 of GenBank Accession No. AE000159.

6. The vector according to any of claims 1-5, wherein said sequence encoding *E.coli ileY* tRNA comprises the sequence between base pairs 7741 and 7950 of GenBank Accession No. AE000350.

7. The vector according to any of claims 1-6, wherein said sequence encoding *E.coli leuW* tRNA comprises the sequence between base pairs 241 and 378 of GenBank Accession No. J01713.

8. A nucleic acid comprising a promoter operably linked to sequences encoding *E.coli argU, ileY* and *leuW* tRNAs.

9. The nucleic acid of claim 8, wherein said sequences encoding *E. coli* tRNAs are present in the order *argU, ileY, leuW.*

10. The nucleic acid according to claim 8 or claim 9, wherein said promoter is the *tet* promoter.

11. The nucleic acid according to any of claims 8-10, wherein the tRNA genes contain their endogenous promoter.

12. The nucleic acid according to any of claims 8-11, wherein said sequence encoding *E.coli argU* tRNA comprises the sequence between base pairs 8041 and 8260 of GenBank Accession No. AE000159.

13. The nucleic acid according to any of claims 8-12, wherein said sequence encoding *E.coli ileY* tRNA comprises the sequence between base pairs 7741 and 7950 of GenBank Accession No. AE000350.

14. The nucleic acid according to any of claims 8-13, wherein said sequence encoding *E.coli leuW* tRNA comprises the sequence between base pairs 241 and 378 of GenBank Accession No. J01713.

15. A host cell comprising the vector of any one of claims 1-7 or the nucleic acid of any one of claims 8-14.

16. The host cell of claim 15, wherein said host cell is an *E.coli* cell.

17. The host cell of claim 16 which has an Hte (high transformation efficiency) phenotype.

18. The host cell of claim 16 which is EndAl deficient.

19. The host cell of claim 16 which is RecA positive.

20. The host cell of claim 16 which has an Hte phenotype and is EndAl deficient, Lon deficient, OmpT deficient, and RecA positive.

21. The host cell of claim 20, wherein transcription of said tRNA genes is controlled by a promoter for T7 RNA polymerase and activated by IPTG.

22. A kit comprising the vector of any one of claims 1-7 or the nucleic acid of any one of claims 8-14, and a host cell, wherein said vector or nucleic acid replicates in said host cell.

## Patentansprüche

1. Ein pACYC-LIC Nukleinsäurevektor umfassend Sequenzen, welche für *E.coli argU, ileY und* /*euW* tRNAs kodieren.

2. Der Vektor gemäß Anspruch 1, wobei die genannten Sequenzen, welche die genannten *E.coli* tRNAs kodieren, in der Reihenfolge argU, ileY, leuW vorhanden sind.

3. Der Vektor gemäß Anspruch 1 oder Anspruch 2, wobei der genannte Vektor einen *tet* Promotor umfasst.

4. Der Vektor nach Anspruch 3, wobei genannter *tet* Promotor die Expression der Sequenz, welche für genannte tRNAs kodiert, antreibt.

5. Der Vektor gemäß einem beliebigen der Ansprüche 1-4, wobei die genannte Sequenz, welche für *E.coli argU* tRNA kodiert, die Sequenz zwischen den Basenpaaren 8041 und 8260 der GenBank Datenbanknummer AE000159 umfasst.

6. Der Vektor gemäß einem beliebigen der Ansprüche 1-5, wobei die genannte Sequenz, welche für *E.coli ileY* tRNA kodiert, die Sequenz zwischen den Basenpaaren 7741 und 7950 der GenBank Datenbanknummer AE000350 umfasst.

7. Der Vektor gemäß einem beliebigen der Ansprüche 1-6, wobei die genannte Sequenz, welche für *E.coli leuW* tRNA kodiert, die Sequenz zwischen den Basenpaaren 241 und 378 der GenBank Datenbanknummer J01713 umfasst.

8. Eine Nukleinsäure umfassend einen Promotor, der funktionell mit Sequenzen, welche für *E.coli argU, ileY und leuW* tRNAs kodieren, verknüpft ist.

9. Die Nukleinsäure nach Anspruch 8, wobei die genannten Sequenzen, welche die genannten *E.coli* tRNAs kodieren, in der Reihenfolge argU, ileY, leuW vorhanden sind.

10. Die Nukleinsäure gemäß Anspruch 8 oder Anspruch 9, wobei der genannte Promotor der *tet* Promotor ist.

11. Die Nukleinsäure gemäß einem beliebigen der Ansprüche 8-10, wobei die tRNA Gene ihren endogenen Promoter enthalten.

12. Die Nukleinsäure gemäß einem beliebigen der Ansprüche 8-11, wobei die genannte Sequenz, welche für *E.coli argU* tRNA kodiert, die Sequenz zwischen den Basenpaaren 8041 und 8260 der GenBank Datenbanknummer AE000159 umfasst.

13. Die Nukleinsäure gemäß einem beliebigen der Ansprüche 8-12, wobei die genannte Sequenz, welche für *E.coli ileY* tRNA kodiert, die Sequenz zwischen den Basenpaaren 7741 und 7950 der GenBank Datenbanknummer AE000350 umfasst.

14. Die Nukleinsäure gemäß einem beliebigen der Ansprüche 8-13, wobei die genannte Sequenz, welche für *E.coli leuW* tRNA kodiert, die Sequenz zwischen den Basenpaaren 241 und 378 der GenBank Datenbanknummer J01713 umfasst.

15. Eine Wirtszelle, welche den Vektor gemäß einem beliebigen der Ansprüche 1-7, oder die Nukleinsäure gemäß einem beliebigen der Ansprüche 8-14 umfasst.

16. Die Wirtszelle nach Anspruch 15, wobei die genannte Wirtszelle eine *E.coli* Zelle ist.

17. Die Wirtszelle nach Anspruch 16, welche einen Hte (hohe Transformationseffizienz) Phänotyp besitzt.

18. Die Wirtszelle nach Anspruch 16, welche EndAl defizient ist.

19. Die Wirtszelle nach Anspruch 16, welche RecA positiv ist.

20. Die Wirtszelle nach Anspruch 16, welche einen Hte (hohe Transformationseffizienz) Phänotyp besitzt und EndAl defizient ist, Lon defizient ist, OmpT defizient ist, und RecA positiv ist.

21. Die Wirtszelle nach Anspruch 20, wobei die Transkription der genannten tRNA Gene von einem Promotor der T7 RNA Polymerase kontrolliert und durch IPTG aktiviert wird.

22. Ein Kit umfassend den Vektor gemäß einem beliebigen der Ansprüche 1-7, oder die Nukleinsäure gemäß einem beliebigen der Ansprüche 8-14, und eine Wirtszelle, wobei der genannte Vektor oder die genannte Nukleinsäure in der genannten Wirtszelle repliziert.

## Revendications

1. Vecteur d'acide nucléique pACYC-LIC comprenant des séquences codant les ARNt d'*E. coli argU, ileY et leuW.*

2. Vecteur selon la revendication 1, dans lequel lesdites séquences codant lesdits ARNt d'*E*. *coli* sont présents dans l'ordre argU, ileY, leuW.

3. Vecteur selon la revendication 1 ou la revendication 2, lequel vecteur comprend un promoteur *tet*.

4. Vecteur selon la revendication 3, dans lequel ledit promoteur *tet* dirige l'expression de la séquence codant lesdits ARNt.

5. Vecteur selon l'une quelconque des revendications 1 à 4, dans lequel ladite séquence codant l'ARNt d'*E*. *coli argU* comprend la séquence entre les paires de bases 8041 et 8260 de numéro d'accès GenBank AE000159.

6. Vecteur selon l'une quelconque des revendications 1 à 5, dans lequel ladite séquence codant l'ARNt d'*E. coli ileY* comprend la séquence entre les paires de bases 7741 et 7950 de numéro d'accès GenBank AE000350.

7. Vecteur selon l'une quelconque des revendications 1 à 6, dans lequel ladite séquence codant l'ARNt d'*E*. *coli leuW* comprend la séquence entre les paires de bases 241 et 378 de numéro d'accès GenBank J01713.

8. Acide nucléique comprenant un promoteur lié de façon opérationnelle à des séquences codant les ARNt d'*E*. *coli argU, ileY et leuW.*

9. Acide nucléique selon la revendication 8, dans lequel lesdites séquences codant les ARNt d'*E*. *coli* sont présents dans l'ordre *argU, ileY, leuW.*

10. Acide nucléique selon la revendication 8 ou la revendication 9, dans lequel ledit promoteur est le promoteur *tet*.

11. Acide nucléique selon l'une quelconque des revendications 8 à 10, dans lequel les gènes d'ARNt contiennent leur promoteur endogène.

12. Acide nucléique selon l'une quelconque des revendications 8 à 11, dans lequel ladite séquence codant l'ARNt d'*E. coli argU* comprend la séquence entre les paires de bases 8041 et 8260 de numéro d'accès GenBank AE000159.

13. Acide nucléique selon l'une quelconque des revendications 8 à 12, dans lequel ladite séquence codant l'ARNt d'*E. coli ileY* comprend la séquence entre les paires de bases 7741 et 7950 de numéro d'accès GenBank AE000350.

14. Acide nucléique selon l'une quelconque des revendications 8 à 13, dans lequel ladite séquence codant l'ARNt d'*E. coli leuW* comprend la séquence entre les paires de bases 241 et 378 de numéro d'accès GenBank J01713.

15. Cellule hôte comprenant le vecteur de l'une quelconque des revendications 1 à 7, ou l'acide nucléique selon l'une quelconque des revendications 8 à 14.

16. Cellule hôte selon la revendication 15, laquelle cellule hôte est une cellule d'E. coli.

17. Cellule hôte selon la revendication 16, qui a un phénotype Hte (efficacité de transformation élevée).

18. Cellule hôte selon la revendication 16, qui est EndAI déficiente.

19. Cellule hôte selon la revendication 16, qui est RecA positive.

20. Cellule hôte selon la revendication 16, qui a un phénotype Hte et est EndAI déficiente, Lon déficiente, OmpT déficiente, et RecA positive.

21. Cellule hôte selon la revendication 20, dans laquelle la transcription desdits gènes d'ARNt est contrôlée par un promoteur pour la T7 ARN polymérase et est activée par l'IPTG.

22. Kit comprenant le vecteur de l'une quelconque des revendications 1 à 7, ou l'acide nucléique selon l'une quelconque des revendications 8 à 14, et une cellule hôte, dans lequel ledit vecteur ou ledit acide nucléique se réplique dans ladite cellule hôte.
